# FASCICULE DE BREVET EUROPEEN

(11) **EP 4 366 802 B1**
(45) Date de publication et mention de la délivrance du brevet: **03.09.2025**
(21) Numéro de dépôt: 22750861.1
(22) Date de dépôt: 04.07.2022
(51) Int. Cl.: A61M 11/00, A61M 15/00, A61M 15/08, B05B 11/02, A61M 11/02, B05B 11/00, B05B 11/06

(54) **DISPOSITIF DE DISTRIBUTION NASALE DE PRODUIT FLUIDE**
VORRICHTUNG ZUR NASALEN VERABREICHUNG EINES FLÜSSIGEN PRODUKTS
DEVICE FOR NASAL DELIVERY OF A FLUID PRODUCT

(30) Priorité: 05.07.2021 FR 2107264
(43) Date de publication de la demande: 15.05.2024
(73) Titulaire: APTAR France SAS, 27110 Le Neubourg (FR)
(72) Inventeur: BAILLET, Matthieu, 76000 Rouen (FR); DU BOISBAUDRY, Guillaume, 76230 Bois Guillaume (FR)
(74) Mandataire: CAPRI
(86) Numéro de dépôt international: PCT/FR2022/051324
(87) Numéro de publication internationale: WO 2023/281194

(56) Documents cités:
- EP-B1- 0 969 891
- WO-A1-2020/154182
- US-A1- 2020 121 871
- US-A1- 2020 197 633

## Description

La présente invention concerne un dispositif de distribution nasale de produit fluide, et plus particulièrement un dispositif pour distribuer dans une narine une dose de produit fluide pharmaceutique, notamment de liquide ou de poudre, contenue dans un réservoir, à l'aide d'un écoulement de gaz sous pression.

Les documents WO9946055 et WO0245866 divulguent des dispositifs de distribution nasale dans lesquels un élément de fermeture sphérique, qui obture la sortie du réservoir, est expulsé par l'écoulement d'air créé par une chasse d'air actionnée manuellement par l'utilisateur. Un inconvénient de ce type de dispositif est la chasse d'air manuelle, qui peut dans l'exemple du document WO9946055 délivrer un écoulement d'air variable selon la manière dont le dispositif est actionné. Le document WO0245866 résout ce problème en proposant une ouverture mécanique en fin de course d'actionnement, mais pour garantir un écoulement d'air suffisant, l'actionnement de la chasse d'air peut s'avérer difficile pour des personnes affaiblies.

Le document WO2020154182 propose de remplacer la chasse d'air manuelle par un réservoir de gaz comprimé qui est ouvert lors de l'actionnement pour distribuer la dose de produit fluide. Le dispositif comporte une aiguille à double pointe, et lorsque l'utilisateur appuie sur le fond du réservoir, l'aiguille va percer d'une part le réservoir de produit fluide et d'autre part le réservoir de gaz comprimé, permettant ainsi au gaz comprimé de distribuer la dose de produit fluide. Cette mise en œuvre présente l'inconvénient d'avoir à exercer une force axiale alors que le dispositif est dans la narine de l'utilisateur, avec des risques d'inconfort ou de blessure lors du déclenchement du flux de gaz comprimé.

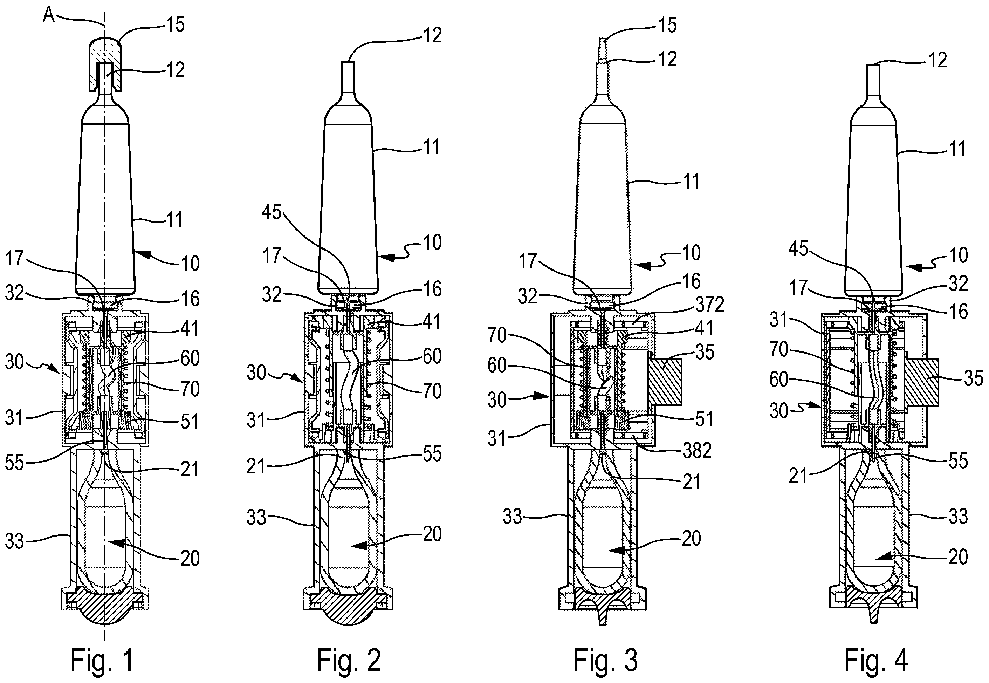

Le document US2020121871 décrit un autre dispositif de l'état de la technique. Il divulgue un dispositif de distribution de produit fluide (fig.1b) comportant un réservoir (123, fig.1b) contenant une dose de produit fluide à distribuer, un réservoir de gaz comprimé (121, fig.1b) adapté à distribuer une dose de gaz comprimé pour expulser la dose de produit fluide lors de l'actionnement, et une partie intermédiaire (100, fig.1b) comportant un corps (111, fig.1b) et des moyens de liaison pour relier ledit réservoir de produit fluide audit réservoir de gaz comprimé, et des moyens d'actionnement (112, fig.1b, para [0017]) pour actionner ledit dispositif, lesdits moyens de liaison comportant un premier organe de support (125, fig.1b) axialement déplaçable par rapport audit corps (fig.2b).

La présente invention a pour but de fournir un dispositif de distribution nasale de produit fluide qui ne reproduit pas les inconvénients susmentionnés.

La présente invention a notamment pour but de fournir un tel dispositif qui garantisse l'expulsion de la dose de produit fluide, indépendamment de la vitesse ou de la force d'actionnement.

La présente invention a aussi pour but de fournir un tel dispositif qui empêche tout risque de blessure dans la narine lors de l'actionnement.

La présente invention a aussi pour but de fournir un tel dispositif qui soit simple et peu coûteux à fabriquer, à assembler, à remplir et à utiliser.

La présente a donc pour objet un dispositif de distribution de produit fluide comportant un réservoir contenant une dose de produit fluide à distribuer, un réservoir de gaz comprimé adapté à distribuer une dose de gaz comprimé pour expulser la dose de produit fluide lors de l'actionnement, et une partie intermédiaire comportant un corps et des moyens de liaison pour relier ledit réservoir de produit fluide audit réservoir de gaz comprimé, et des moyens d'actionnement pour actionner ledit dispositif, lesdits moyens de liaison comportant un premier organe de support et un second organe de support axialement déplaçables par rapport audit corps, et un tube souple et déformable fixé d'une part audit premier organe de support et d'autre part audit second organe de support, ledit premier organe de support comportant des premiers moyens d'ouverture pour, en position d'actionnement, ouvrir ledit réservoir de produit fluide, et ledit second organe de support comportant des seconds moyens d'ouverture pour, en position d'actionnement, ouvrir ledit réservoir de gaz comprimé, un ressort chargé étant disposé entre lesdits premier et second organes de support, pour les solliciter élastiquement vers leur position d'actionnement respective, lesdits moyens d'actionnement comportant un bouton d'actionnement déplaçable radialement entre une position de repos et une position d'actionnement, ledit bouton d'actionnement étant solidaire d'une cage creuse qui, en position de repos dudit bouton d'actionnement, bloque axialement lesdits organes de support dans leurs positions de repos respectives, et lorsque ledit bouton d'actionnement est déplacé radialement vers sa position d'actionnement, lesdits organes de support sont axialement débloqués et ledit ressort déplace lesdits deux organes de support vers leurs positions d'actionnement respectives.

Selon une première variante avantageuse, ledit réservoir de produit fluide contient une dose unique de produit fluide.

Avantageusement, lesdits premiers moyens d'ouverture comportent une première aiguille, reliée audit tube souple, et qui, en position d'actionnement, perce une membrane dudit réservoir de produit fluide.

Selon une première variante avantageuse, ledit réservoir de gaz comprimé contient une dose unique de gaz comprimé.

Avantageusement, lesdits seconds moyens d'ouverture comportent une seconde aiguille, reliée audit tube souple, et qui, en position d'actionnement, perce une membrane dudit réservoir de gaz comprimé.

Selon une seconde variante avantageuse, ledit réservoir de gaz comprimé contient plusieurs doses de gaz comprimé.

Avantageusement, lesdits seconds moyens d'ouverture comportent un puits de soupape, relié audit tube souple, et qui coopère avec une soupape d'une valve doseuse montée sur ledit réservoir de gaz comprimé.

Avantageusement, ladite cage creuse comporte une paroi axiale supérieure et une paroi axiale inférieure, chaque paroi axiale comportant des parties de paroi et des ouvertures axiales.

Avantageusement, en position de repos, un bord axial supérieur dudit premier organe de support est axialement bloqué par une partie de paroi de ladite paroi axiale supérieure, et un bord axial inférieur dudit second organe de support est axialement bloqué par une partie de paroi de ladite paroi axiale inférieure.

Avantageusement, lorsque ledit bouton d'actionnement est déplacé radialement vers sa position d'actionnement, une ouverture axiale de ladite paroi axiale supérieure se positionne face audit bord axial supérieur dudit premier organe de support, et une ouverture axiale de ladite paroi axiale inférieure se positionne face audit bord axial inférieur dudit second organe de support.

Avantageusement, en position de repos, ledit tube souple est déformé et/ou tordu, et lorsque lesdits deux organes de support se déplacent axialement l'un par rapport à l'autre vers leurs positions d'actionnement respectives, ledit tube souple se redresse, pour compenser l'écartement axial généré entre les deux organes de support lors de l'actionnement.

Avantageusement, lors de l'actionnement, ledit réservoir de produit fluide et ledit réservoir de gaz comprimé sont ouverts simultanément.

En variante, lors de l'actionnement, ledit réservoir de produit fluide est ouvert juste avant ledit réservoir de gaz comprimé.

Avantageusement, ledit réservoir est du type BFS ("Blow-Fill-Seal") ou FFS ("Form-Fill-Seal"), c'est-à-dire réalisé par soufflage ou formage, remplissage et scellage en continu.

Avantageusement, ledit réservoir comporte des moyens de fixation pour fixer ledit réservoir à ladite partie intermédiaire, notamment de manière amovible.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section transversale verticale d'un dispositif de distribution de produit fluide selon un premier mode de réalisation avantageux, avant actionnement,
- la figure 2 est une vue similaire à celle de la figure 1, après actionnement,
- les figures 3 et 4 sont des vues similaires à celles des figures 1 et 2, vues selon un angle différent,
- la figure 5 est une vue schématique de détail en section transversale verticale du dispositif de la figure 3,
- la figure 6 est une vue schématique de détail en section transversale verticale du dispositif de la figure 4,
- la figure 7 est une vue schématique de dessus en section transversale horizontale de la partie intermédiaire, avant actionnement,
- la figure 8 est une vue similaire à celle de la figure 7, après actionnement,
- la figure 9 est une vue schématique en perspective d'un dispositif de distribution de produit fluide selon un second mode de réalisation avantageux, avant actionnement, et
- la figure 10 est une vue schématique en perspective découpée du dispositif de la figure 9, après actionnement.

Il est entendu que dans la description ci-après, les termes "supérieur", "inférieur", "haut", "bas", "vertical" et "horizontal" se réfèrent à la position droite du dispositif représenté sur les figures 1 à 4, 9 et 10. Les termes "axial" et "radial" se réfèrent à l'axe central longitudinal A du dispositif, représenté sur la figure 1.

Le dispositif comporte trois parties principales, à savoir un réservoir 10 contenant une dose de produit fluide à distribuer, en particulier un médicament sous forme de poudre ou de liquide, un réservoir de gaz comprimé 20 adapté à distribuer une dose de gaz comprimé lors de l'actionnement pour expulser la dose de produit fluide lors de l'actionnement, et une partie intermédiaire 30 reliant le réservoir 10 au réservoir de gaz comprimé 20 et permettant l'actionnement du dispositif.

Le réservoir 10 peut être formé directement dans une tête de distribution 11 de forme axialement allongée, dont l'extrémité axiale comporte un orifice de distribution 12, et qui est destinée à être insérée dans la narine d'un utilisateur, comme représenté dans les exemples des figures. En variante, le réservoir pourrait être séparé de la tête de distribution.

Dans un mode de réalisation préféré, visible sur les figures, le réservoir 10 est du type BFS ("Blow-Fill-Seal") ou FFS ("Form-Fill-Seal"), c'est-à-dire que le réservoir 10 est réalisé par soufflage ou formage, remplissage et scellage en continu, de préférence dans une enceinte stérile.

L'orifice de distribution 12 est fermé par un bouchon amovible 15 qui est retiré juste avant l'utilisation du dispositif. Après retrait du bouchon 15, l'orifice de distribution 12 est ouvert.

Du côté axialement opposé à l'orifice de distribution 12, le réservoir 10 est fermé par une membrane 17 adaptée à être ouverte, notamment percée, lors de l'actionnement, comme cela sera décrit ci-après.

Avantageusement, le réservoir 10 comporte des moyens de fixation 16 pour fixer le réservoir 10 à la partie intermédiaire 30. Cette fixation peut être indémontable, lorsque le dispositif est à usage unique et jetable après actionnement. En variante, la fixation peut être démontable, pour remplacer un réservoir vide par un réservoir plein, lorsque le dispositif est réutilisable. Un exemple d'une fixation démontable serait une fixation rotative du type Luer, bien connue dans le domaine des seringues.

Dans le premier mode de réalisation des figures 1 à 6, le réservoir de gaz comprimé 20 contient une seule dose de gaz comprimé. Dans ce cas, le réservoir de gaz comprimé 20 peut être indémontable de la partie intermédiaire 30 si le dispositif est à usage unique et jetable après utilisation. En variante, le réservoir de gaz comprimé 20 pourrait être démontable pour remplacer un réservoir de gaz 20 vide par un réservoir de gaz 20 plein, lorsque le dispositif est réutilisable.

Dans ce premier mode de réalisation, le réservoir de gaz comprimé 20 est fermé par une membrane 21 adaptée à être ouverte, notamment percée, lors de l'actionnement, comme cela sera décrit ci-après.

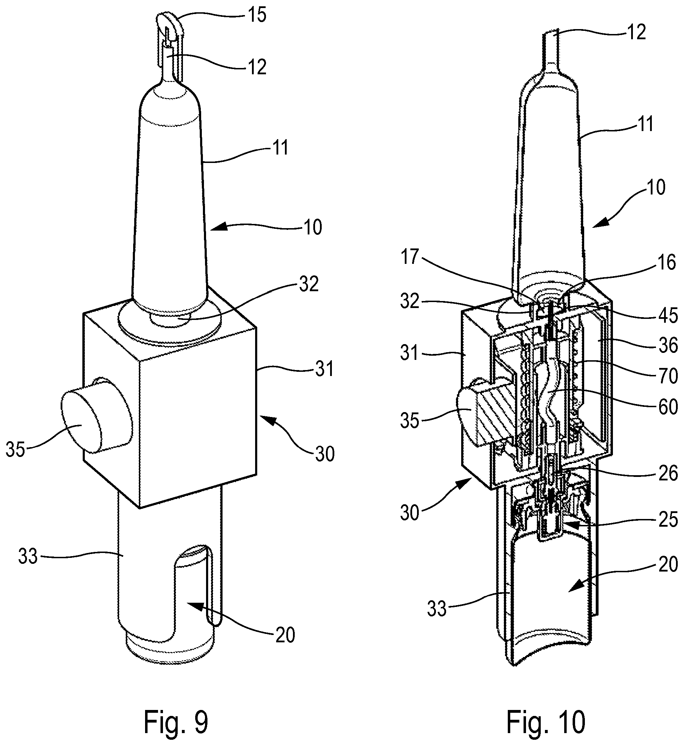

Dans le second mode de réalisation des figures 9 et 10, le réservoir de gaz comprimé 20 contient plusieurs doses de gaz comprimé.

Dans ce cas, le réservoir de gaz comprimé 20 comporte de préférence une valve doseuse 25 comportant une soupape 26 axialement déplaçable dans la valve lors de l'actionnement pour distribuer une dose de gaz comprimé. L'ouverture du réservoir de gaz comprimé 20 se produit alors lorsque la soupape 26 est déplacée vers sa position actionnée, en étant axialement enfoncée dans le corps de valve, de manière bien connue. Les valves doseuses fonctionnant avec des gaz comprimés sont bien connues, et la valve doseuse 25, qui peut être de tout type existant, ne sera pas décrite plus amplement ici.

La partie intermédiaire 30 comporte un corps 31 pourvu d'une partie de corps supérieur 32 et d'une partie de corps inférieur 33. La partie de corps supérieur 32 comporte avantageusement des moyens de fixation complémentaires des moyens de fixation 16 du réservoir 10, par exemple une fixation standard du type Luer. La partie de corps inférieur 33 reçoit le réservoir de gaz comprimé 20.

Un bouton d'actionnement 35 est monté radialement coulissant dans le corps 31 entre une position de repos et une position d'actionnement dans laquelle le bouton d'actionnement 35 est enfoncé radialement vers l'intérieur du corps 31. Le bouton d'actionnement 35 est solidaire d'une cage creuse 36.

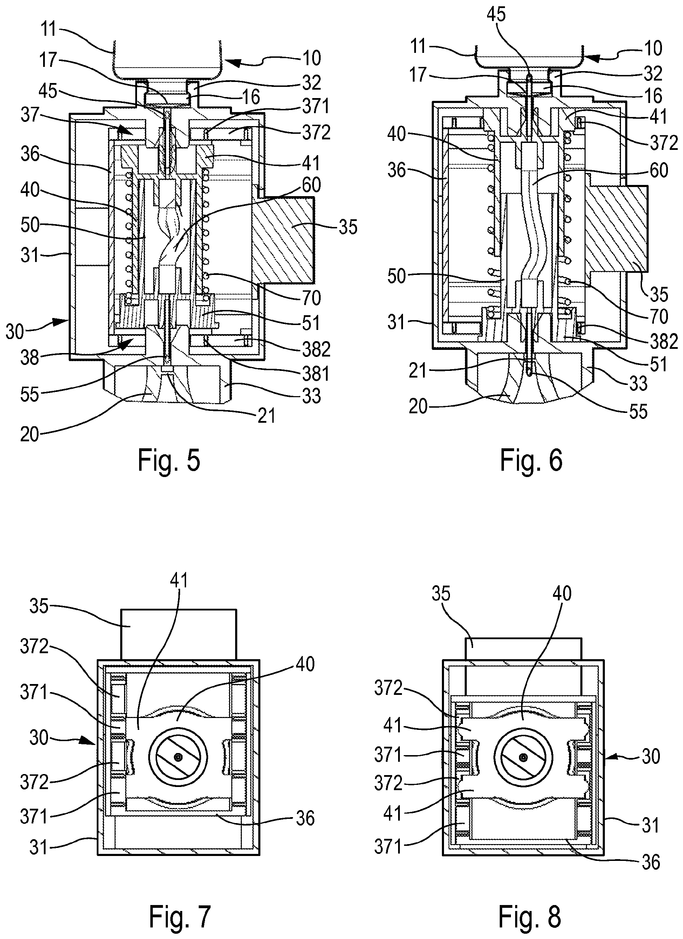

Avantageusement, la cage creuse 36 comporte une paroi axiale supérieure 37 et une paroi axiale inférieure 38, chaque paroi axiale 37, 38 comportant des parties de parois 371, 381 et des ouvertures axiales 372, 382, visibles sur les figures 5 à 8.

Le corps 31 de la partie intermédiaire 30 contient un premier organe de support 40 et un second organe de support 50 disposés l'un autour de l'autre en étant chacun axialement déplaçable par rapport au corps 31. Le premier organe de support 40 est déplaçable axialement vers le haut entre sa position de repos et sa position d'actionnement, et le second organe de support 50 est déplaçable axialement vers le bas entre sa position de repos et sa position d'actionnement.

Le premier organe de support 40 comporte un bord axial supérieur 41 et le second organe de support 50 comporte un bord axial inférieur 51.

Un tube souple et déformable 60 est fixé d'une part au premier organe de support 40 et d'autre part au second organe de support 50. En position de repos, ce tube souple est déformé et/ou tordu, et lorsque les deux organes de support 40, 50 se déplacent axialement l'un par rapport à l'autre vers leurs positions d'actionnement respectives, le tube souple 60 se redresse, permettant ainsi de compenser l'écartement axial généré entre les deux organes de support 40, 50 lors de l'actionnement.

Le premier organe de support 40 comporte des premiers moyens d'ouverture 45 du réservoir de produit fluide 10. Dans les exemples représentés, ces premiers moyens d'ouvertures comportent une première aiguille 45, reliée audit tube souple 60, et qui, en position d'actionnement, perce la membrane 17 du réservoir 10.

Le second organe de support 50 comporte des seconds moyens d'ouverture 55 du réservoir de gaz comprimé 20. Dans l'exemple représenté sur les figures 1 à 6, ces seconds moyens d'ouvertures comportent une seconde aiguille 55, reliée audit tube souple 60, et qui, en position d'actionnement, perce la membrane 21 du réservoir de gaz comprimé 20.

Ainsi, en position d'actionnement, le contenu du réservoir de gaz comprimé 20 est relié au contenu du réservoir de produit fluide 10 via les deux aiguilles 55, 45 et le tube souple 60, assurant la distribution dudit produit fluide à travers l'orifice de distribution 12.

Un ressort 70 chargé est disposé entre lesdits premier et second organes de support 40, 50, pour les solliciter élastiquement vers leur position d'actionnement respective.

La cage creuse 36, en position de repos du bouton d'actionnement 35, bloque lesdits organes de support 40, 50 dans leurs positions de repos, et lorsque le bouton d'actionnement 35 est déplacé radialement vers sa position d'actionnement, les organes de support 40, 50 sont débloqués et le ressort 70 déplace les deux organes de support 40, 50 vers leurs positions d'actionnement respectives. Ainsi, l'actionnement du bouton d'actionnement latéral 35 provoque simultanément l'ouverture du réservoir de produit fluide et l'ouverture du réservoir de gaz comprimé 20. Eventuellement, notamment en adaptant les positions respectives des deux organes de support 40, 50, il est possible de faire en sorte que lors de l'actionnement, le réservoir de produit fluide 10 soit ouvert juste avant l'ouverture du réservoir de gaz comprimé 20.

Les propriétés du ressort 70, notamment sa raideur et/ou la force qu'il exerce lors de l'actionnement sur les deux organes de support 40, 50, permettent de prédéterminer les performances du spray généré en sortie de l'orifice de distribution 12.

D'autres paramètres influent aussi sur ces performances du spray, notamment le volume et la pression de la dose de gaz comprimé délivrée lors de l'actionnement, ainsi que le volume du produit fluide contenu dans le réservoir 10.

Avantageusement, comme représenté sur la figure 7, en position de repos, le bord axial supérieur 41 du premier organe de support 40 bute contre une partie de paroi 371 de la paroi axiale supérieure 37 de la cage 36 et le bord axial inférieur 51 du second organe de support 50 bute contre une partie de paroi 381 de la paroi axiale inférieure 38 de la cage 36.

Lorsque le bouton d'actionnement 35 est déplacé radialement vers sa position d'actionnement, représentée sur la figure 8, une ouverture axiale 372 de la paroi axiale supérieure 37 de la cage 36 vient se positionner face audit bord axial supérieur 41 du premier organe de support 40, et une ouverture axiale 382 de la paroi axiale inférieure 38 de la cage 36 vient se positionner face audit bord axial inférieur 51 du second organe de support 50. Les deux organes de support 40, 50 ne sont alors plus bloqués axialement, et le ressort 70 déplace chaque organe de support 40, 50 vers sa position d'actionnement respective.

Lorsque l'utilisateur souhaite utiliser le dispositif, il retire d'abord le bouchon 15 de l'orifice de distribution 12 du réservoir 10, et place la tête de distribution dans une narine. Il appuie alors sur le bouton d'actionnement 35, ce mouvement d'actionnement étant latéral et non axial, pour déplacer la cage 36 radialement dans le corps 31 et libérer axialement les deux organes de support 40, 50. Le ressort 70 réalise alors automatiquement l'actionnement et la distribution de la dose de produit fluide contenue dans le réservoir 10 au moyen du gaz comprimé contenu dans le réservoir de gaz comprimé 20.

Après utilisation, le dispositif est jeté, s'il est à usage unique.

En variante, on peut envisager de réutiliser la partie intermédiaire 30, en remplaçant le réservoir de produit fluide 10 vide par un nouveau réservoir de produit fluide 10 et en remplaçant le réservoir de gaz comprimé 20 vide par un nouveau réservoir de gaz comprimé 20. Dans ce cas, le bouton d'actionnement 35 comporte avantageusement des moyens de réarmement pour le ramener en position de repos après chaque actionnement. Avantageusement, ces moyens de réarmement comportent un élément élastique, tel qu'un second ressort. De préférence, la force exercée par ce second ressort est supérieure à la force exercée par le ressort 70, de sorte que le réarmement se fait automatiquement, en ramenant d'une part le bouton d'actionnement 35 vers sa position de repos et en ramenant également les deux organes de support 40, 50 vers leurs positions de repos respectives, en rechargeant le ressort 70.

Dans le second mode de réalisation des figures 9 et 10, on utilise un réservoir de gaz comprimé 20 contenant plusieurs doses de gaz comprimé, le dispositif étant alors réutilisable, avec changement de réservoir 10 après chaque actionnement.

Dans ce second mode de réalisation, le second organe de support 50 ne comporte pas d'aiguille pour percer une membrane, mais coopère avec la soupape 26 de la valve doseuse 25 montée sur le réservoir de gaz comprimé 20. Ainsi, à chaque actionnement, une dose de gaz comprimé est délivrée pour distribuer le produit fluide contenu dans le réservoir 10.

Le dispositif étant réutilisable, le bouton d'actionnement 35 comporte des moyens de réarmement pour le ramener en position de repos après chaque actionnement. Avantageusement, ces moyens de réarmement comportent un élément élastique, tel qu'un second ressort. De préférence, la force exercée par ce second ressort est supérieure à la force exercée par le ressort 70, de sorte que le réarmement se fait automatiquement, en ramenant d'une part le bouton d'actionnement 35 vers sa position de repos et en ramenant également les deux organes de support 40, 50 vers leurs positions de repos respectives, en rechargeant le ressort 70.

Le dispositif selon l'invention améliore la distribution du produit fluide dans la narine, en permettant au produit d'atteindre des parties plus lointaines, telles que les éthmoïdes.

Le dispositif selon l'invention est notamment adapté pour être utilisé dans le traitement des rhinites allergiques et du syndrome de sensibilisation centrale.

Dans le cadre d'une rhinite allergique, le produit fluide peut être choisi dans la liste suivante, donnée à titre d'exemple non limitatif : fluticasone, mometasone, fluticasone furoate, budésonide, béclometasone, azelastine fluticasone, azelastine, tixocortol, triamcinolone acetonide, ipratropium bromide, prednisolone, ciclesonide, flunisolide, levocabastine, azelastine fluticasone furoate, olopatadine, azelastine mometasone, mometasone olopatadine, fluticasone olopatadine.

Dans le cadre d'un syndrome de sensibilisation centrale, le produit fluide peut être choisi dans la liste suivante, donnée à titre d'exemple non limitatif : sumatriptan, zolmitriptan, naloxone, fentanyl, esketamine, midazolam, butorphanol, dihydroergotamine, diazépam, tapentadol, alfentanil, diamorphine, ketorolac.

La présente invention a été décrite en référence à divers modes et variantes de réalisation, mais il est entendu qu'un homme du métier peut y apporter toutes modifications, sans sortir du cadre de la présente invention telle que défini par les revendications annexées.

## Revendications

1. Dispositif de distribution de produit fluide comportant un réservoir (10) contenant une dose de produit fluide à distribuer, un réservoir de gaz comprimé (20) adapté à distribuer une dose de gaz comprimé pour expulser la dose de produit fluide lors de l'actionnement, et une partie intermédiaire (30) comportant un corps (31) et des moyens de liaison (40, 50, 60) pour relier ledit réservoir de produit fluide (10) audit réservoir de gaz comprimé (20), et des moyens d'actionnement (35, 36) pour actionner ledit dispositif, lesdits moyens de liaison comportant un premier organe de support (40) et un second organe de support (50) axialement déplaçables par rapport audit corps (31), et un tube souple et déformable (60) fixé d'une part audit premier organe de support (40) et d'autre part audit second organe de support (50), ledit premier organe de support (40) comportant des premiers moyens d'ouverture (45) pour, en position d'actionnement, ouvrir ledit réservoir de produit fluide (10), et ledit second organe de support (50) comportant des seconds moyens d'ouverture (55) pour, en position d'actionnement, ouvrir ledit réservoir de gaz comprimé (20), un ressort (70) chargé étant disposé entre lesdits premier et second organes de support (40, 50), pour les solliciter élastiquement vers leur position d'actionnement respective, lesdits moyens d'actionnement comportant un bouton d'actionnement (35) déplaçable radialement entre une position de repos et une position d'actionnement, ledit bouton d'actionnement (35) étant solidaire d'une cage creuse (36) qui, en position de repos dudit bouton d'actionnement (35), bloque axialement lesdits organes de support (40, 50) dans leurs positions de repos respectives, et lorsque ledit bouton d'actionnement (35) est déplacé radialement vers sa position d'actionnement, lesdits organes de support (40, 50) sont axialement débloqués et ledit ressort (70) déplace lesdits deux organes de support (40, 50) vers leurs positions d'actionnement respectives.

2. Dispositif selon la revendication 1, dans lequel ledit réservoir de produit fluide contient une dose unique de produit fluide.

3. Dispositif selon la revendication 1 ou 2, dans lequel lesdits premiers moyens d'ouverture (45) comportent une première aiguille, reliée audit tube souple (60), et qui, en position d'actionnement, perce une membrane (17) dudit réservoir de produit fluide (10).

4. Dispositif selon l'une quelconque des revendications précédentes, dans lequel ledit réservoir de gaz comprimé (20) contient une dose unique de gaz comprimé.

5. Dispositif selon la revendication 4, dans lequel lesdits seconds moyens d'ouverture (55) comportent une seconde aiguille, reliée audit tube souple (60), et qui, en position d'actionnement, perce une membrane (21) dudit réservoir de gaz comprimé (20).

6. Dispositif selon l'une quelconque des revendications 1 à 3, dans lequel ledit réservoir de gaz comprimé (20) contient plusieurs doses de gaz comprimé.

7. Dispositif selon la revendication 6, dans lequel lesdits seconds moyens d'ouverture (55) comportent un puits de soupape, relié audit tube souple (60), et qui coopère avec une soupape (26) d'une valve doseuse (25) montée sur ledit réservoir de gaz comprimé (20).

8. Dispositif selon l'une quelconque des revendications précédentes, dans lequel ladite cage creuse (36) comporte une paroi axiale supérieure (37) et une paroi axiale inférieure (38), chaque paroi axiale (37, 38) comportant des parties de paroi (371, 381) et des ouvertures axiales (372, 382).

9. Dispositif selon la revendication 8, dans lequel, en position de repos, un bord axial supérieur (41) dudit premier organe de support (40) est axialement bloqué par une partie de paroi (371) de ladite paroi axiale supérieure (37), et un bord axial inférieur (51) dudit second organe de support (50) est axialement bloqué par une partie de paroi (381) de ladite paroi axiale inférieure (38).

10. Dispositif selon la revendication 9, dans lequel, lorsque ledit bouton d'actionnement (35) est déplacé radialement vers sa position d'actionnement, une ouverture axiale (372) de ladite paroi axiale supérieure (37) est configurée pour se positionner face audit bord axial supérieur (41) dudit premier organe de support (40), et une ouverture axiale (382) de ladite paroi axiale inférieure (38) est configurée pour se positionner face audit bord axial inférieur (51) dudit second organe de support (50).

11. Dispositif selon l'une quelconque des revendications précédentes, dans lequel, en position de repos, ledit tube souple (60) est déformé et/ou tordu, et lorsque lesdits deux organes de support (40, 50) se déplacent axialement l'un par rapport à l'autre vers leurs positions d'actionnement respectives, ledit tube souple (60) est configuré pour se redresser, pour compenser l'écartement axial généré entre les deux organes de support (40, 50) lors de l'actionnement.

12. Dispositif selon l'une quelconque des revendications précédentes, dans lequel, lors de l'actionnement, ledit réservoir de produit fluide (10) et ledit réservoir de gaz comprimé (20) sont configurés pour être ouverts simultanément.

13. Dispositif selon l'une quelconque des revendications 1 à 11, dans lequel, lors de l'actionnement, ledit réservoir de produit fluide (10) est configuré pour être ouvert juste avant ledit réservoir de gaz comprimé (20).

14. Dispositif selon l'une quelconque des revendications précédentes, dans lequel ledit réservoir (10) est du type BFS ("Blow-Fill-Seal") ou FFS ("Form-Fill-Seal"), c'est-à-dire réalisé par soufflage ou formage, remplissage et scellage en continu.

15. Dispositif selon l'une quelconque des revendications précédentes, dans lequel ledit réservoir (10) comporte des moyens de fixation (16) pour fixer ledit réservoir (10) à ladite partie intermédiaire (30), notamment de manière amovible.

## Patentansprüche

1. Vorrichtung zur Verabreichung eines fluiden Produkts, die einen Vorratsbehälter (10) aufweist, der eine Dosis eines zu verabreichenden fluiden Produkts enthält, einen Druckgasbehälter (20), der geeignet ist, bei Betätigung eine Dosis Druckgas abzugeben, um die Dosis des fluiden Produkts auszustoßen, und einen Zwischenteil (30), der einen Körper (31) und Verbindungsmittel (40, 50, 60) zum Verbinden des Vorratsbehälters für das fluide Produkt (10) mit dem Druckgasbehälter (20) und Betätigungsmittel (35, 36) zum Betätigen der Vorrichtung aufweist, wobei die Verbindungsmittel ein erstes Halteorgan (40) und ein zweites Halteorgan (50), die axial in Bezug auf den Körper (31) verlagerbar sind, und einen flexiblen und verformbaren Schlauch (60) aufweisen, der einerseits an dem ersten Halteorgan (40) und andererseits an dem zweiten Halteorgan (50) befestigt ist, wobei das erste Halteorgan (40) erste Öffnungsmittel (45) aufweist, um in Betätigungsstellung den Vorratsbehälter (10) für das fluide Produkt zu öffnen, und das zweite Halteorgan (50) zweite Öffnungsmittel (55) aufweist, um in Betätigungsstellung den Druckgasbehälter (20) zu öffnen, wobei eine vorgespannte Feder (70) zwischen dem ersten und dem zweiten Halteorgan (40, 50) angeordnet ist, um diese elastisch in ihre jeweilige Betätigungsstellung zu beanspruchen, wobei die Betätigungsmittel einen Betätigungsknopf (35) aufweisen, der radial zwischen einer Ruhestellung und einer Betätigungsstellung verlagerbar ist, wobei der Betätigungsknopf (35) mit einem hohlen Käfig (36) fest verbunden ist, der in Ruhestellung des Betätigungsknopfs (35) die Halteorgane (40, 50) in ihren jeweiligen Ruhestellungen axial blockiert, und, wenn der Betätigungsknopf (35) radial in seine Betätigungsstellung verlagert wird, die Halteorgane (40, 50) axial freigegeben werden und die Feder (70) die beiden Halteorgane (40, 50) in ihre jeweiligen Betätigungspositionen verlagert.

2. Vorrichtung nach Anspruch 1, wobei der Vorratsbehälter für das fluide Produkt eine Einzeldosis des fluiden Produkts enthält.

3. Vorrichtung nach Anspruch 1 oder 2, wobei die ersten Öffnungsmittel (45) eine erste Nadel aufweisen, die mit dem flexiblen Schlauch (60) verbunden ist und die in Betätigungsstellung eine Membran (17) des Vorratsbehälters (10) für das fluide Produkt durchsticht.

4. Vorrichtung nach einem der vorstehenden Ansprüche, wobei der Druckgasbehälter (20) eine Einzeldosis Druckgas enthält.

5. Vorrichtung nach Anspruch 4, wobei die zweiten Öffnungsmittel (55) eine zweite Nadel umfassen, die mit dem flexiblen Schlauch (60) verbunden ist und in die in Betätigungsstellung eine Membran (21) des Druckgasbehälters (20) durchsticht.

6. Vorrichtung nach einem der Ansprüche 1 bis 3, wobei der Druckgasbehälter (20) mehrere Dosen Druckgas enthält.

7. Vorrichtung nach Anspruch 6, wobei die zweiten Öffnungsmittel (55) einen mit dem flexiblen Schlauch (60) verbundenen Ventilschacht aufweisen, und der mit einem Ventil (26) eines an dem Druckgasbehälter (20) angebrachten Dosierventils (25) zusammenwirkt.

8. Vorrichtung nach einem der vorstehenden Ansprüche, wobei der hohle Käfig (36) eine obere axiale Wand (37) und eine untere axiale Wand (38) aufweist, wobei jede axiale Wand (37, 38) Wandabschnitte (371, 381) und axiale Öffnungen (372, 382) aufweist.

9. Vorrichtung nach Anspruch 8, wobei in Ruhestellung eine obere axiale Kante (41) des ersten Halteorgans (40) durch einen Wandabschnitt (371) der oberen axialen Wand (37) axial blockiert ist und eine untere axiale Kante (51) des zweiten Halteorgans (50) durch einen Wandabschnitt (381) der unteren axialen Wand (38) axial blockiert ist.

10. Vorrichtung nach Anspruch 9, wobei, wenn der Betätigungsknopf (35) radial in seine Betätigungsposition verlagert wird, eine axiale Öffnung (372) der oberen axialen Wand (37) ausgelegt ist, um sich gegenüber der oberen axialen Kante (41) des ersten Halteorgans (40) zu positionieren, und eine axiale Öffnung (382) der unteren axialen Wand (38) ausgelegt ist, um sich gegenüber der unteren axialen Kante (51) des zweiten Halteorgans (50) zu positionieren.

11. Vorrichtung nach einem der vorstehenden Ansprüche, wobei in Ruhestellung der flexible Schlauch (60) verformt und/oder verdreht ist und wenn sich die beiden Halteorgane (40, 50) relativ zueinander in ihre jeweiligen Betätigungspositionen axial verlagern, der flexible Schlauch (60) ausgelegt ist, um sich zu strecken, um den bei der Betätigung zwischen den beiden Halteorganen (40, 50) entstehenden axialen Abstand auszugleichen.

12. Vorrichtung nach einem der vorstehenden Ansprüche, wobei bei Betätigung der Vorratsbehälter (10) für das fluide Produkt und der Druckgasbehälter (20) ausgelegt sind, um gleichzeitig geöffnet zu werden.

13. Vorrichtung nach einem der Ansprüche 1 bis 11, wobei bei Betätigung der Vorratsbehälter (10) für das fluide Produkt ausgelegt ist, um unmittelbar vor dem Druckgasbehälter (20) geöffnet zu werden.

14. Vorrichtung nach einem der vorstehenden Ansprüche, wobei der Vorratsbehälter (10) vom Typ BFS ("Blow-Fill-Seal") oder FFS ("Form-Fill-Seal") ist, das heißt, durch kontinuierliches Blasformen oder Form-, Füll- und Siegelverfahren hergestellt ist.

15. Vorrichtung nach einem der vorstehenden Ansprüche, wobei der Vorratsbehälter (10) Befestigungsmittel (16) zum insbesondere lösbaren Befestigen des Vorratsbehälters (10) am Zwischenteil (30) aufweist.

## Claims

1. Device for dispensing fluid product, comprising a vessel (10) containing a dose of fluid product to be dispensed, a compressed gas vessel (20) suitable for dispensing a dose of compressed gas to expel the dose of fluid product during actuation, and an intermediate portion (30) comprising a body (31) and connection means (40, 50, 60) for connecting said fluid product vessel (10) to said compressed gas vessel (20), and actuating means (35, 36) for actuating said device, said connection means comprising a first support member (40) and a second support member (50) movable axially with respect to said body (31), and a flexible and deformable tube (60) attached both to said first support member (40) and to said second support member (50), said first support member (40) comprising first opening means (45), for, in the actuating position, opening said fluid product vessel (10), and said second support member (50) comprising second opening means (55) for, in the actuating position, opening said compressed gas vessel (20), a loaded spring (70) being disposed between said first and second support members (40, 50), for elastically urging them to their respective actuating position, said actuating means comprising an actuating button (35) movable radially between a rest position and an actuating position, said actuating button (35) being rigidly connected to a hollow cage (36) which, in the rest position of said actuating button (35), locks said support members (40, 50) axially in their respective rest positions, and when said actuating button (35) is moved radially to its actuating position, said support members (40, 50) are unlocked axially and said spring (70) moves said two support members (40, 50) to their respective actuating positions.

2. Device according to claim 1, wherein said fluid product vessel contains a single dose of fluid product.

3. Device according to claim 1 or 2, wherein said first opening means (45) comprise a first needle, connected to said flexible tube (60), and which, in the actuating position, pierces a membrane (17) of said fluid product vessel (10).

4. Device according to any one of the preceding claims, wherein said compressed gas vessel (20) contains a single dose of compressed gas.

5. Device according to claim 4, wherein said second opening means (55) comprise a second needle, connected to said flexible tube (60), and which, in the actuating position, pierces a membrane (21) of said compressed gas vessel (20).

6. Device according to any one of claims 1 to 3, wherein said compressed gas vessel (20) contains several doses of compressed gas.

7. Device according to claim 6, wherein said second opening means (55) comprise a valve well, connected to said flexible tube (60), and which engages with a valve (26) of the dosing valve (25) mounted on said compressed gas vessel (20).

8. Device according to any one of the preceding claims, wherein said hollow cage (36) comprises an upper axial wall (37) and a lower axial wall (38), each axial wall (37, 38) comprising wall portions (371, 381) and axial openings (372, 382).

9. Device according to claim 8, wherein, in the rest position, an upper axial edge (41) of said first support member (40) is locked axially by a wall portion (371) of said upper axial wall (37), and a lower axial edge (51) of said second support member (50) is locked axially by a wall portion (381) of said lower axial wall (38).

10. Device according to claim 9, wherein, when said actuating button (35) is moved radially to its actuating position, an axial opening (372) of said upper axial wall (37) is configured to be positioned facing said upper axial edge (41) of said first support member (40), and an axial opening (382) of said lower axial wall (38) is configured to be positioned facing said lower axial edge (51) of said second support member (50).

11. Device according to any one of the preceding claims, wherein, in the rest position, said flexible tube (60) is deformed and/or twisted, and when said two support members (40, 50) move axially relative to one another to their respective actuating positions, said flexible tube (60) is configured to straighten, to compensate for the axial gap generated between the two support members (40, 50) during actuation.

12. Device according to any one of the preceding claims, wherein, during actuation, said fluid product vessel (10) and said compressed gas vessel (20) are configured to be opened simultaneously.

13. Device according to any one of claims 1 to 11, wherein, during actuation, said fluid product vessel (10) is configured to be opened just before said compressed gas vessel (20).

14. Device according to anyone of the preceding claims, wherein said vessel (10) is of the BFS (Blow-Fill-Seal) or FFS (Form-Fill-Seal) type, i.e. produced by blowing or forming, filling and sealing continuously.

15. Device according to any one of the preceding claims, wherein said vessel (10) comprises attachment means (16) for attaching said vessel (10) to said intermediate portion (30), in particular, removably.
